# EUROPEAN PATENT APPLICATION

(11) **EP 4 483 820 A1**
(43) Date of publication of application: **01.01.2025**
(21) Application number: 24185253.2
(22) Date of filing: 28.06.2024
(51) Int. Cl.: A61B 17/16

(54) **PERFORATOR**

(30) Priority: 30.06.2023 GB 202310087
(71) Applicant: Adeor Medical AG, 83626 Valley (DE)
(72) Inventor: HASBACH, Dominic, 83626 Valley (DE); VON ZEPPELIN, Fabio, 83626 Valley (DE)
(74) Representative: Reddie & Grose LLP

(57) **Abstract**

A perforator for drilling bone tissue is provided. The perforator comprises a drive shaft with a rotational axis A; an inner cutting head with the same rotational axis A, configured to cut a substantially cylindrical hole; and a coupling mechanism for coupling the drive shaft and the inner cutting head. The inner cutting head comprises an engaging side wall portion having a radius r1, the radius r1 being a maximum radius of the inner cutting head so that radius r1 defines a radius of a hole the inner cutting head is configured to cut, and a further side wall portion having a radius which is smaller than radius r1. An inner cutting head is also provided.

## Description

The present invention relates to a perforator for cutting a hole in bone tissue. In a particularly preferred embodiment the invention relates to a perforator for cutting a hole in a skull of a human or animal.

### Background

In order to carry out surgical operations inside the cranial cavity, it is necessary to first obtain access to the cranial cavity by drilling one or more holes through the bone of the skull (the cranium). The process of drilling a hole through the skull is called trepanation. Trepanation is a difficult and delicate procedure, as sufficient force must be applied to the cutting head for it to advance through the hard layers of bone tissue, but the cutting head must be halted immediately after perforation of the skull in order to avoid the cutting head damaging the dura mater or soft tissue inside the cranium.

Prior art perforators have typically been similar to conventional drills, in that a forward force or pressure must be applied to the drill to drive the cutting head through the skull. A major risk with such prior art designs is that when the cranium is perforated, the forward force or pressure on the cutting head can drive the cutting head inwards through the newly created hole and into the cranium, potentially causing damage to the dura mater.

Attempts to reduce this risk of damage to the dura mater have focused on stopping rotation of the cutting head as soon as the cranium is perforated, so that the rotating cutting or cutting head does not tear or otherwise damage the dura mater. In order to provide a perforator in which the rotation of the cutting head stops as soon as the cranium has been perforated, cranial perforators are provided in the prior art in which the cutting head is not permanently coupled to a drive shaft of an electric drill.

Known examples of prior art designs are disclosed in US Patent No. 4,456,010 and International Patent Application No. WO 2015/150844. The drill of US 4,456,010 comprises an inner cutting head, an outer cutting head arranged coaxially around the inner cutting head, and a drive shaft, all rotatable about the same axis of rotation. The inner cutting head is biased away from the drive shaft with a spring, so that when there is no pressure applied to the tip of the inner cutting head, the cutting head and the shaft are disconnected, and neither of the inner or outer cutting heads rotates even when the drive shaft is rotating. Connection of the inner cutting head to the drive shaft is controlled using a slot-and-pin type clutch that comprises a slot in the distal end of the drive shaft, and a corresponding pin in the proximal end of the cutting head. The clutch-pin also extends into a triangular slot in the wall of the outer cutting head.

As the inner cutting head of US 4,456,010 is pressed against bone in use, a force is applied which moves the inner cutting head against the biasing spring until the clutch-pin in the inner cutting head engages with the slot in the rotating drive shaft. In this position, the clutch-pin also extends into a triangular slot in the wall of the outer cutting head and engages with the apex of the triangular slot, so that rotational force is transmitted from the drive shaft via the inner cutting head to the outer cutting head, and both cutting heads rotate together. As long as sufficient force is applied to the tip of the inner cutting head to overcome the biasing force of the spring such that the clutch-pin remains engaged with the slot, the rotating drive shaft of the electric drill transmits rotational force to the inner and outer cutting heads so that both cutting heads rotate and the perforator drills through the bone. As soon as the inner cutting head perforates the inner surface of the cranium, however, force is no longer applied to the cutting head by the bone, so the biasing spring urges the inner cutting head forward and forces the clutch-pin out of the slot in the drive shaft. Both the inner and outer cutting heads should then cease to rotate immediately, to prevent damage to the dura mater.

As sufficient force is applied to the tip of the inner cutting head to overcome the biasing force of the spring for the perforator to drill bone, for the clutch to disengage, the skull must be perforated.

The prior art design of WO 2015/150844 replaces the slot-and-pin clutch of US 4,456,010 with two square-sided first connecting profiles on the proximal end of the cutting head, and two square-sided second connecting profiles on the distal end of the drive shaft. Nevertheless, this prior art design also requires sufficient force to be applied to the tip of the inner cutting head to overcome the biasing force of the spring.

The penetration depth of prior art perforators is up to 5 mm, most commonly 3 or 4 mm. This penetration depth, and failure of the release mechanism to separate immediate, causes dura or brain injuries in about 1 % of trepanations. Additionally, prior art perforators are highly complex, and to secure safe cooperation of the various components, high process reliability is required, which increases costs.

The inventors have appreciated the need for a perforator in which there is a reduced risk of damage to anything underlying the bone tissue being cut by the perforator, or in which the risk is eliminated completely.

### Summary of Invention

The disclosure provides a perforator for drilling bone tissue, and an inner cutting head for a perforator for drilling bone tissue, as defined in the appended independent claims, to which reference should now be made. Optional or advantageous features of the disclosure are set out in dependent subclaims.

In a first aspect, the disclosure provides a perforator for drilling bone tissue, comprising: a drive shaft with a rotational axis A; an inner cutting head with the same rotational axis A; and a coupling mechanism for coupling the drive shaft and the inner cutting head. The inner cutting head comprises an engaging side wall portion having a radius r1, the radius r1 being a maximum radius of the inner cutting head, and a further side wall portion having a radius which is smaller than radius r1.

The inner cutting head is configured to cut a substantially circular, or cylindrical, hole. As the inner cutting head rotates around the rotational axis A in use, radius r1 defines a radius of the hole which the inner cutting head is configured to cut. The rotation of the engaging side wall portion of the inner head bores a circular hole into bone tissue in use, which forms a cylindrical hole as the hole proceeds through a thickness of bone tissue, and the bone tissue surrounding the bored hole forms a cylindrical side wall to the hole.

In conventional perforators, the side walls of the inner cutting head have a uniform outer radius along their length, so as the inner cutting head rotates and bores a hole into bone tissue, the radially-outermost side walls are in constant contact with the cylindrical side wall formed by the bone tissue surrounding the hole.

In the present perforator, the inner cutting head has a side wall which comprises two portions: an engaging side wall portion, and a further side wall portion. The engaging side wall portion acts as the radially-outermost surface of the inner cutting head as it rotates, so the engaging side wall portion contacts the cylindrical wall of bone tissue surrounding the hole while the perforator is drilling into the bone tissue. The further side wall portion, however, is narrower than the engaging side wall portion, meaning that the further side wall portion is not in contact with the bone tissue surrounding the hole while the perforator is drilling into the bone tissue.

In the present perforator, because the side wall of the inner cutting head is only partially in contact with the bone tissue defining the boundary of the hole being cut, a contact area between the side wall of the inner cutting head and the bone tissue defining the boundary of the hole being cut is reduced when compared to a prior art inner cutting head.

The reduced contact area between the inner cutting head and the bone tissue may advantageously reduce friction between the inner cutting head and the bone being cut. This may in turn result in less drive power being required to rotate the inner cutting head, reducing wear on the coupling mechanism. It may also reduce the force required for removing the perforator from the skull once a hole has been drilled, and may further result in reduced heat generation during drilling.

Where reference is made to the "bone tissue defining the boundary of the hole", this refers to the bone tissue which surrounds the hole which is being cut into the bone tissue by the perforator.

As used herein the term "distal" refers to portions of the perforator positioned towards the tip of the perforator, which is intended to cut the bone tissue in use, while the term "proximal" refers to portions of the perforator that are, in use, further from the bone being drilled and closer to, for example, the hand of a user holding a drill.

The inner cutting head preferably comprises a side wall, the side wall comprising the engaging side wall portion and the further side wall portion.

In a first embodiment, the engaging side wall portion may occupy discrete axial positions along the side wall of the inner cutting head. The engaging side wall portion may be positioned distally relative to the further side wall portion. As the perforator is proceeding into bone tissue, the wider engaging side wall portion thus cuts the hole into the bone tissue, with the radius r1 of the engaging side wall portion defining the radius of the resulting hole. The further side wall portion, proximal to the engaging side wall portion, then follows the engaging side wall portion into the hole that is being cut, with the narrower radius of the further side wall portion ensuring that the further side wall portion is not in contact with the bone tissue surrounding the hole.

In a second embodiment, the engaging side wall portion and the further side wall portion may occupy the same axial positions along the side wall of the inner cutting head, but discrete radial positions around the side wall of the inner cutting head. For example at a single axial position on the cutting head, one or more engaging side wall portions may be arranged around the circumference of the inner cutting head side wall, with the one or more engaging side wall portions being separated by one or more further side wall portions.

The engaging side wall portion is the portion of the inner cutting head side wall which engages, or contacts, the bone tissue forming the cylindrical sides of the hole which is cut by the perforator. The engaging side wall portion may alternatively be named a first side wall portion.

The further side wall portion may alternatively be named a narrowed side wall portion of the inner cutting head.

The radius r1 may be greater than the radius of the further side wall portion by about 0.005 mm to about 5 mm, or about 0.01 mm to about 1 mm, or about 0.015 mm to about 0.5 mm, or about 0.02 mm to about 0.1 mm, or about 0.025 mm to about 0.05 mm.

Although a specific difference in radius between radius r1 and the radius of the further side wall portion is defined above, in some embodiments, the further side wall portion may have a varying radius. The difference between the radius r1 and the radius of the further side wall portion may be a minimum difference between the radius r1 and the radius of the further side wall portion.

In some embodiments, the further side wall portion has a radius r2 which is less than the radius r1 of the engaging side wall portion.

The radius r1 may be greater than the radius r2 of the further side wall portion by about 0.005 mm to about 5 mm, or about 0.01 mm to about 1 mm, or about 0.015 mm to about 0.5 mm, or about 0.02 mm to about 0.1 mm, or about 0.025 mm to about 0.05 mm.

The inventors have found, surprisingly, that a difference in radius of only a few hundredths of a millimetre may significantly reduce the contact area between the side wall of the inner cutting head and the bone tissue.

Optionally, the inner cutting head further comprises a widening forming at least a portion of the engaging side wall portion.

In other words, the inner cutting head may further comprise a widened section, or widening section. The widening, or widening section, or widened section, may have a constant radius along a length (in an axial direction) of the widening, or a radius of the widening may vary along a length (in an axial direction) of the widening (or widening section, or widened section).

As used herein, the term "widening" (or "Aufweitung") may refer to a feature which increases a radius of the cutting head, relative to the radius of the further side wall portion. The widening, which may alternatively be referred to as a widened section, or a widening section, or a radial extension, of the inner cutting head is wider than the further side wall portion, meaning that it has a radius greater than the further side wall portion. The widening, or widened section, may take a variety of shapes, and may encompass a taper, a bulge, and stepped, or abrupt, increases in radius, e.g. steps.

Advantageously, by providing a widening forming at least a portion of the engaging side wall portion, a risk of the inner cutting head jamming in the hole being cut may be reduced or eliminated.

The widening preferably has rotational symmetry about the axis A. Advantageously, the widening may allow for cutting of a hole having a uniform radius.

It is noted that the widening (or widened section, or widening section) having rotational symmetry about the axis A may comprise multiple portions distributed around a circumference of the inner cutting head. The multiple portions may be arranged uniformly around the circumference of the inner cutting head so that the widening has rotation symmetry. The widening may, for example, not be continuous, i.e. there may be circumferential gaps in the widening. In one embodiment, there may be four widening portions forming the widening, the widening portions separated by circumferential gaps of equal size. In another embodiment, the widening may be continuous around the circumference of the inner cutting head, thus forming an annular widening.

Optionally, the circumferential gaps between the widening portions extend a total of 100 degrees to about 300 degrees, or 150 degrees to about 280 degrees, or 200 degrees to 270 degrees, or 250 degrees to 260 degrees, about the circumference of the inner cutting head.

The widening portions may extend a total of 50 degrees to about 150 degrees, or 70 degrees to about 130 degrees, or 90 degrees to about 110 degrees, about the circumference of the inner cutting head.

Each widening portion may extend 10 degrees to 50 degrees, or about 20 degrees to about 30 degrees, or about 25 degrees, about the circumference of the inner cutting head.

Each circumferential gap may extend about 40 degrees to about 80 degrees, or about 60 degrees to about 70 degrees, or about 65 degrees, about the circumference of the inner cutting head.

The widening may have a thickness (a difference in radius across the widening) which is equal to the difference between the radius r1 and the radius of the further side wall portion.

It has been surprisingly found by the inventors that a widening having a thickness (or, expressed differently, a difference in radius relative to the further side wall portion) of only a few hundredths of a millimetre may significantly reduce the contact area between the side wall of the inner cutting head and the bone tissue defining the boundary of the hole.

The widening may be arranged at a distal end portion of the inner cutting head, so that the engaging side wall portion is arranged distally of the further side wall portion.

Advantageously, as the engaging side wall portion defines a radius of a hole being cut, providing the widening at a distal end portion of the inner cutting head may allow for the hole to be cut by a distal end portion of the inner cutting head, permitting the more proximal parts of the inner cutting head to not be in contact with the bone tissue defining the hole already cut. In embodiments in which the inner cutting head is configured to be biased into the proximal position, this means that there is less friction acting against the inner cutting head being biased into the proximal position when the inner cutting head has cut through the bone tissue, thus reducing the risk of the inner cutting head jamming in the hole.

Optionally, the widening has a length, along the rotational axis A, of less than about 5 mm, or less than about 2 mm, or less than about 1 mm, optionally of about 0.1 mm to about 1 mm, or about 0.5 mm to about 0.9 mm, or about 0.8 mm.

The inventors have found, surprisingly, that even a widening having a length of less than 1 mm may significantly reduce the contact area between the side walls of the inner cutting head and the bone tissue.

As such, given the relatively small thickness and/or length of the widening, very little additional material may be required to achieve the reduced contact area.

It is noted that, while a length of "the widening" is defined, this may refer to each of the widening portions forming the widening. That is, each widening portion may have a length, along the rotational axis A, as defined above.

The widening may comprise a stepped widening. Alternatively or additionally, the widening may comprise a tapered widening.

Advantageously, a stepped widening may be easier to manufacture, and may ensure that a contact area between the inner cutting head and bone tissue may be reduced.

If a substantially annular (i.e. continuous), tapered widening is provided, then a portion of the inner cutting head comprising the annular, tapered widening may be substantially frustoconical.

If the widening is a tapered widening as discussed below, the radius r1 may refer to a maximum radius of the widening.

The inner cutting head may comprise at least one cutting protrusion. Preferably, the inner cutting head comprises at least two cutting protrusions. Optionally, the at least two cutting protrusions are provided with cutting edge flutes. The cutting head flutes may allow for materials to flow away from the distal end of the cutting head.

It is noted that inner cutting heads of the prior art may already comprise cutting edge flutes. However, in contrast to the prior art cutting heads, in the inner cutting head according to the present disclosure, for example according to the first aspect, only a portion of the part of the side wall associated with each cutting protrusion (the "engaging side wall portion") engages with the bone tissue forming the boundary of the hole being cut. The remaining portion of the part of the side wall associated with each cutting protrusion (the "further side wall portion"), is not in contact with the bone tissue forming the boundary of the hole being cut. In other words, the further side wall portion according to the disclosure may refer only to the "substantially cylindrical portions" of the side wall, and does not relate to the cutting edge flutes for allowing material flow away from the distal end of the cutting head.

The inner cutting head, at a distal end, may further comprise a plurality of cutting protrusions. Advantageously, providing a plurality of cutting protrusions allows for a hole to be cut more efficiently, and for the cutting properties of the cutting head to be tuned more precisely. Optionally, each of the plurality of cutting protrusions comprises a widening portion forming a part of the, or a, widening. Advantageously, by providing a widening portion on each of the cutting protrusions, the engaging side wall portion may define a more even circumference for defining the radius of the substantially circular hole being cut. In fact, if the cutting protrusions are provided evenly around the circumference of the cutting head, this may allow for the widening to be rotationally symmetrical about axis A.

In some embodiments, the inner cutting head further comprises: a helical groove extending around the side wall of the inner cutting head along the rotational axis A; and a margin forming at least a portion of the engaging side wall portion. Optionally, the helical groove comprises a plurality of flutes. Further optionally, a margin is provided adjacent each flute.

The term "margin" refers to a section of the side wall that remains between helical grooves. The "margin" is a portion of the side wall of the inner cutting head that is not cut away, and thus defines the largest radius (of the circumference) of the inner cutting head.

In other words, in some embodiments, the inner cutting head may comprise a margin, which is not cut away and thus defines the maximum radius r1 of the circumference of the inner cutting head (and the radius of the hole being cut), making up at least a portion of the engaging side wall portion. In these embodiments, the further side wall portion may be made up, at least partially, of the flutes of the helical groove, as the flutes of the groove have radii which are less than the radius r1 of the margins defining the engaging side wall potion of the inner cutting head.

Advantageously, providing a margin reduces the contact area between the inner cutting head and the bone tissue defining the boundary of the hole being cut.

Optionally, the margin has a width of less than about 3 mm, or less than about 2 mm, or less than about 1 mm. Advantageously, by providing a narrow margin, a contact area of the inner cutting head with the bone tissue defining a boundary of the hole being cut is reduced.

At least a portion of the inner cutting head may be substantially frustoconical. In some embodiments, a frustoconical portion of the inner cutting head may form the tapered widening, with the widest part of the frustoconical portion defining the radius r1. In some embodiments, the entire inner cutting head is substantially frustoconical. Preferably, the frustoconical portion of the inner cutting head widens in a distal direction, such that a distal end of the frustoconical portion is wider than a proximal end of the frustoconical portion.

It is noted that an inner cutting head may comprise a combination of a widening, a frustoconical portion, and/or a helical groove and a margin.

Preferably, the inner cutting head is displaceable with respect to the drive shaft along the rotational axis between a distal position, in which the inner cutting head is not driveable by the drive shaft, and a proximal position, in which the coupling mechanism couples the drive shaft and the inner cutting head to transmit rotational motion from the drive shaft to the inner cutting head.

Depending on the cutting geometry of cutting heads, patient anatomy, and specific surgical procedures, prior art cutting heads may become jammed in the hole being cut. This may lead to a delayed uncoupling of the inner cutting head from the drive shaft, meaning that the inner cutting head rotates longer than it is intended to, which may increase a risk to the patient.

The perforator according to the first aspect having an inner cutting head having an engaging side wall portion and a further side wall portion, resulting in a reduced contact area between the inner cutting head side wall and bone tissue defining a boundary of the hole being cut, in which the inner cutting head is displaceable between a non-drivable distal position, and a drivable proximal position, may allow for the risk of the inner cutting head jamming in the hole being cut to be significantly reduced, or to be avoided altogether.

Optionally, the perforator further comprises a biasing means for biasing the inner cutting head into the distal position. Preferably, the biasing means is a spring.

Further, a perforator according to the first aspect in which the inner cutting head is displaceable between a non-drivable distal position, and a drivable proximal position, which further comprises a widening may allow for decoupling of the inner cutting head to be more noticeable. The decoupling may be more noticeable audibly and/or tactilely.

In some embodiments, the perforator further comprises an outer cutting head arranged coaxially around the inner cutting head, in which a cutting head coupling mechanism is provided, the cutting head coupling mechanism being configured to couple the inner cutting head and the outer cutting head when the inner cutting head is in the proximal position so that rotational motion is transmitted from the inner cutting head to the outer cutting head.

Advantageously, by providing an outer cutting head selectively coupleable to the inner cutting head, the perforator may more easily cut a hole of a suitable size, while allowing the inner cutting head to securely decouple to increase patient safety.

Optionally, the coupling mechanism comprises the cutting head coupling mechanism. Advantageously, if the coupling mechanism comprises the cutting head coupling mechanism, the two coupling mechanisms may share common components, thus reducing complexity and mitigating the risk of breakage.

The perforator may be configured so that when the inner cutting head is in the proximal position, a distal end portion of the inner cutting head projects, distally, beyond a distal end of the outer cutting head. The distal end portion which projects beyond the outer cutting head preferably comprises at least a portion of each of the engaging side wall portion and the further side wall portion.

Optionally, the further side wall portion makes up at least 10 % of a side wall of the distal end portion of the inner cutting head projecting distally beyond the distal end of the outer cutting head when the inner cutting head is in the proximal position, optionally wherein the further side wall portion makes up at least 20%, at least 30%, at least 40%, at least 50%, at least 60%, at least 70%, or at least 80%, or at least 90%, or at least 95%, or at least 99%, of the projecting side wall.

Advantageously, if the further side wall portion makes up at least 10 % of a side wall of the distal end portion of the inner cutting head projecting distally beyond the distal end of the outer cutting head, a contact area between the side wall of the inner cutting head and bone tissue defining the boundary of a hole in the bone tissue may be reduced significantly.

The perforator may preferably be configured so that when the inner cutting head is in the proximal position, the engaging side wall portion and the further side wall portion of the inner cutting head extend distally beyond a distal end of the outer cutting head.

Optionally, the further side wall portion makes up at least 10 % of a side wall of the portion of the inner cutting head which projects distally beyond the outer cutting head when the inner cutting head is in the distal position, optionally wherein the further side wall portion makes up at least 20%, at least 30%, at least 40%, at least 50%, at least 60%, at least 70%, or at least 80%, or at least 90%, or at least 95%, or at least 99%, of the projecting side wall.

Advantageously, if the further side wall portion makes up at least 10 % of a side wall of the inner cutting head projecting distally beyond the distal end of the outer cutting head when the inner cutting head is in the distal position, a contact area between the side wall of the inner cutting head and bone tissue defining the boundary of a hole in the bone tissue is reduced significantly, while the inner cutting head is being biased from the proximal position into the distal position. This means that there is less friction acting against the inner cutting head being biased into the proximal position when the inner cutting head has cut through the bone tissue, thus reducing the risk of the inner cutting head jamming in the hole.

The cutting head coupling mechanism may be configured to transform relative rotation of the inner and outer cutting heads around the rotational axis A into translational displacement of the inner cutting head along the rotational axis. Preferably, the cutting head coupling mechanism comprises an angled edge configured to transform relative rotation of the inner and outer cutting heads into translational displacement of the inner cutting head along the rotational axis.

Optionally, the outer cutting head comprises an outer cutting head engaging side wall portion having a radius r3, the radius r3 being a maximum radius of the outer cutting head, and an outer cutting head further side wall portion having a radius which is smaller than radius r3.

The outer cutting head is configured to cut a substantially circular, or cylindrical, hole. As the outer cutting head rotates around the rotational axis A in use, radius r3 defines a radius of the hole which the inner cutting head is configured to cut. The rotation of the outer cutting head engaging side wall portion of the outer cutting head bores a circular hole into bone tissue in use, which forms a cylindrical hole as the hole proceeds through a thickness of bone tissue, and the bone tissue surrounding the bored hole forms a cylindrical side wall to the hole.

Advantageously, in this way a contact area between the outer cutting head, and the bone tissue defining the boundary of a hole being cut by the outer cutting head is reduced.

Preferably, at least a portion of the outer cutting head is substantially frustoconical.

According to a second aspect of the present disclosure, there is provided an inner cutting head for a perforator for cutting bone tissue according to any of the preceding claims, comprising: an engaging side wall portion having a radius r1, the radius r1 being a maximum radius of the cutting head so that radius r1 defines a radius of a hole the cutting head is configured to cut; and a further side wall portion having a radius which is smaller than radius r1.

The inner cutting head is configured to cut a substantially circular, or cylindrical, hole. As the inner cutting head rotates around the rotational axis A in use, radius r1 defines a radius of the hole which the inner cutting head is configured to cut. The rotation of the engaging side wall portion of the inner head bores a circular hole into bone tissue in use, which forms a cylindrical hole as the hole proceeds through a thickness of bone tissue, and the bone tissue surrounding the bored hole forms a cylindrical side wall to the hole.

In some embodiments, the inner cutting head further comprises: a coupling mechanism portion configured to engage a corresponding coupling mechanism portion of a drive shaft of a perforator when the cutting head is in a proximal position along a rotational axis of the drive shaft, to transmit rotational motion from the drive shaft to the inner cutting head.

Optionally, the coupling mechanism portion may comprise a drive pin configured to engage a drive surface of the drive shaft.

The radius r1 may be greater than the radius of the further side wall portion by about 0.005 mm to about 5 mm, or about 0.01 mm to about 1 mm, or about 0.015 mm to about 0.5 mm, or about 0.02 mm to about 0.1 mm, or about 0.025 mm to about 0.05 mm.

Although a specific difference in radius between radius r1 and the radius of the further side wall portion is defined above, in some embodiments, the further side wall portion may have a varying radius, and the difference defined above may be a minimum difference between the radius r1 and the radius of the further side wall portion.

The inventors have found, surprisingly, that a difference in radius of only a few hundredths of a millimetre may significantly reduce the contact area between the side wall of the inner cutting head and the bone tissue defining a boundary of the hole being cut.

Optionally, the inner cutting head further comprises a widening forming at least a portion of the engaging side wall portion. The widening preferably has rotational symmetry about the axis A.

Optionally, the widening is arranged at a distal end portion of the inner cutting head, so that the engaging side wall portion is arranged distally of the further side wall portion.

Optionally, the widening has a length, along a rotational axis A of the inner cutting head, of less than about 5 mm, or less than about 2 mm, or less than about 1 mm, optionally of about 0.1 mm to about 1 mm, or about 0.5 mm to about 0.9 mm, or about 0.8 mm.

In some embodiments, the widening comprises a stepped widening, or a tapered widening.

Optionally, at least a portion of the inner cutting head may be substantially frustoconical.

In some embodiments, the inner cutting head, at a distal end, comprises a plurality of cutting protrusions, and preferably each of the plurality of cutting protrusions comprises a widening portion forming a part of the, or a, widening, further preferably of the, or a widening having rotational symmetry about the axis A.

The inner cutting head may further comprise: a helical groove extending along the rotational axis A; and a margin forming at least a portion of the engaging side wall portion. Preferably, the margin has a width of less than about 3 mm, or less than about 2 mm, or less than about 1 mm.

In a further aspect, the disclosure provides a perforator for drilling bone tissue, comprising: a drive shaft with a rotational axis A; an inner cutting head with the same rotational axis A, configured to cut a substantially cylindrical, or cylindrical, hole; and a coupling mechanism for coupling the drive shaft and the inner cutting head, wherein the inner cutting head comprises reducing means configured to reduce a contact area between a side wall of the inner cutting head and bone tissue defining a boundary of the hole being cut. The inner cutting head having a "reduced" contact area may refer to the contact area being smaller than if the side walls of at least the cutting protrusions of the inner cutting head were substantially cylindrical. The reducing means may comprise at least one of: a widening, at least one widening portion, a substantially frustoconical portion and/or a helical groove and a margin.

In a yet further aspect, the disclosure provides a perforator for drilling bone tissue, comprising: a drive shaft with a rotational axis A; an inner cutting head with the same rotational axis A, configured to cut a substantially cylindrical hole; and a coupling mechanism for coupling the drive shaft and the inner cutting head, wherein the inner cutting head is configured so that, in use, a contact area between a side wall of the inner cutting head and bone tissue defining a boundary of the hole being cut is reduced. The inner cutting head may be configured so that a contact area is reduced by being provided with at least one of: a widening, at least one widening portion, a substantially frustoconical portion and/or a helical groove.

Any feature in one aspect of the invention may be applied to other aspects of the invention, in any appropriate combination. Furthermore, any, some and/or all features in one aspect may be applied to any, some and/or all features in any other aspect, in any appropriate combination. In particular, any product features provided in relation to the first aspect may be applied to any of the other aspects. In particular, any of the features disclosed in respect of the perforator of the first aspect may be applied to the perforator of the further aspect, or of the yet further aspect, and to the inner cutting head of the second aspect.

It should also be appreciated that particular combinations of the various features described and defined in any aspects of the invention may be implemented and/or supplied and/or used independently.

### Brief description of drawings

The disclosure will be further described, by way of example only, with reference to the accompanying drawings, in which:
Figure 1 is a partially cut-away side view of a perforator according to the prior art;
Figure 2 is a perspective view of an example of a perforator according to the present disclosure;
Figure 3 is a further perspective view of the example perforator according to the present disclosure;
Figure 4 is an exploded side view of the example perforator according to the present disclosure;
Figure 5 is a sectional side view of the example perforator according to the present disclosure;
Figure 6A is a side view of an example inner cutting head according to the present disclosure;
Figure 6B is a perspective view of the example inner cutting head according to the present disclosure;
Figure 7 is a distal end view of the example inner cutting head according to the present disclosure;
Figure 8 is a partial side view of an alternative example of an inner cutting head according to the present disclosure;
Figure 9A is a side view of a further alternative example of an inner cutting head according to the present disclosure; and
Figure 9B is a distal end view of the further alternative example of an inner cutting head according to the present disclosure.

### Specific description

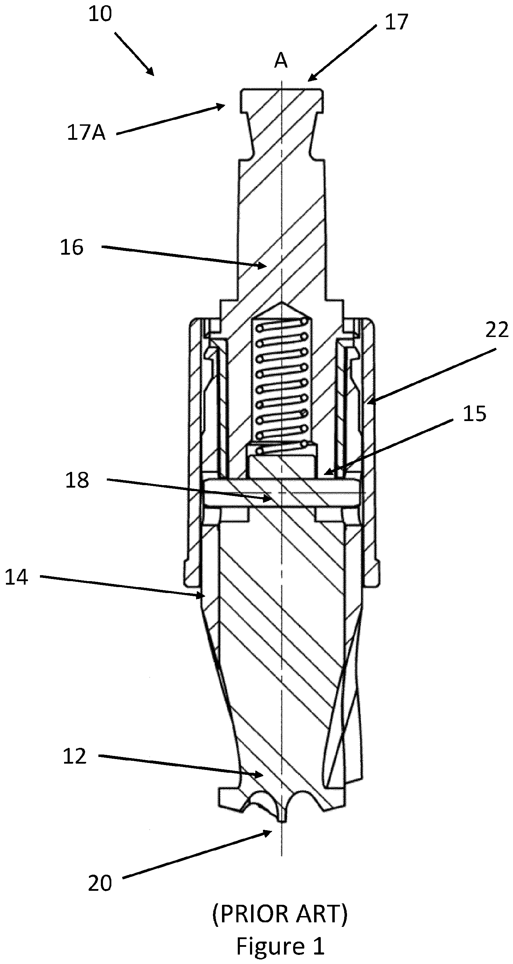

Figures 1 illustrates the parts of a prior art perforator design similar to the perforator disclosed in WO 2015/150844.

In Figure 1, the perforator 10 comprises an inner cutting head 12, a hollow outer chipping head 14 arranged coaxially around the inner cutting head 12, and a drive shaft 16, all rotatable about the same axis of rotation A. The drive shaft has a distal end 15 and a proximal end 17, the proximal end 17 being connectable, for example, to a hand-held drill housing a motor. In particular, the proximal end 17 is a standardised connector 17A, such as a Hudson connector.

In use, the perforator 10 is connected to a manual or, more commonly, an electric drill. The distal end of the perforator 10 terminates in a distal tip 20 of the inner cutting head 12, which contacts bone in use, and is shown at the bottom of Figure 1. The proximal end of the perforator 10 terminates in the standardised connector 17A of the drive shaft 16, which is shown at the top in Figure 1.

The inner cutting head 12 is movable along the rotational axis A between two positions: a distal position, in which the inner cutting head 12 is not connected to the drive shaft 16, and a proximal cutting position, in which the inner cutting head 12 is connected to the drive shaft 16, so as to transmit rotation of the drive shaft 16 to the inner cutting head 12. The inner cutting head is biased away from the drive shaft 16 and into the distal position by a spring 18, so that the inner cutting head only moves into the proximal cutting position when the distal tip 20 of the inner cutting head 12 is pressed against a surface, such as a bone. The force with which the distal tip 20 must be pressed against the surface to overcome the biasing force of the spring 18 is determined by the spring constant of the spring 18 and the axial separation between the distal position and the proximal cutting position.

When no pressure is applied to the distal tip 20 of the inner cutting head 12, the inner cutting head 12 and the drive shaft 16 are disconnected, and neither of the inner cutting head 12 and the outer chipping head 14 rotates even when the drive shaft 16 is rotating. As such, the user must exert a force in a direction towards the surface of the material to be cut so that the inner cutting head 12 is pressed against the surface with sufficient force to overcome the biasing force of the spring. A cylindrical housing 22 is arranged coaxially around the outer chipping head 14.

As soon as the distal tip 20 of the inner cutting head 12 perforates the bone (for example the inner surface of the cranium), the force applied to the tip of the inner cutting head 12 by the bone is greatly reduced, so that it is exceeded by the force exerted by the biasing spring 18 on the inner cutting head 12, so that the biasing spring 18 urges the inner cutting head 12 forward, out of the proximal, cutting, position and into the distal, non-cutting, position. As the biasing spring 18 urges the inner cutting head 12 into its distal position, both the inner and outer cutting heads should cease to rotate immediately to prevent damage to the dura mater.

The present inventors have found, however, that in the prior art designs it cannot be guaranteed that the inner cutting head does not damage the dura mater. This is in part because a large contact area between the inner cutting head and the bone tissue being cut (in particular between the side wall of the inner cutting head, more particularly the "substantially cylindrical" portion of the side wall of the cutting protrusions, and the "inner" walls of bone tissue defining the hole being cut) may cause the inner cutting head to become jammed in the hole being cut. This may lead to a delayed decoupling of the inner cutting head from the drive shaft, meaning that the inner cutting head rotates longer than it is intended to, which may increase a risk to the patient.

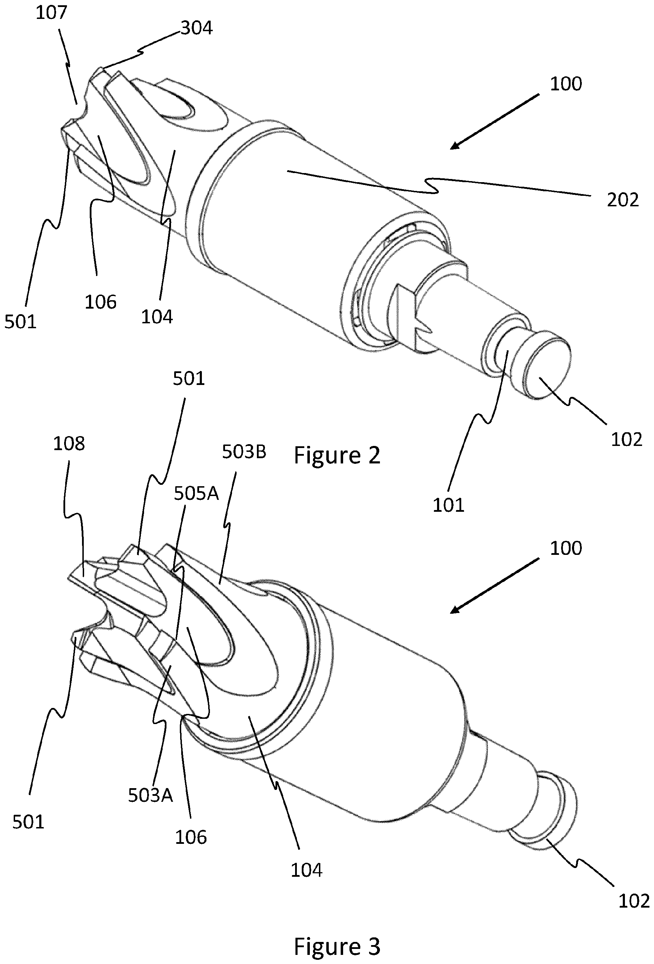

Figures 2 to 7 illustrate a perforator 100 according to the present disclosure. Similar to the prior art perforator 10 of Figure 1, the perforator 100 has a drive shaft 101 having, at a proximal end, a standardised connector 102, such as a Hudson connector. In use, the perforator 100 is connected to a manual drill or, more commonly, an electric drill via the standardised connector 102.

The perforator 100 further comprises an outer cutting head 104, and, arranged coaxially within the outer cutting head 104, an inner cutting head 106. The drive shaft 101, outer cutting head 104 and inner cutting head 106 are all rotatable about the rotational axis A. A distal tip 107 of the inner cutting head 106 forms a distal end of the perforator 100. At the distal end, the inner cutting head 106 comprises a plurality of cutting protrusions 501 terminating in cutting edges 108 for cutting a hole into bone tissue, in particular a skull.

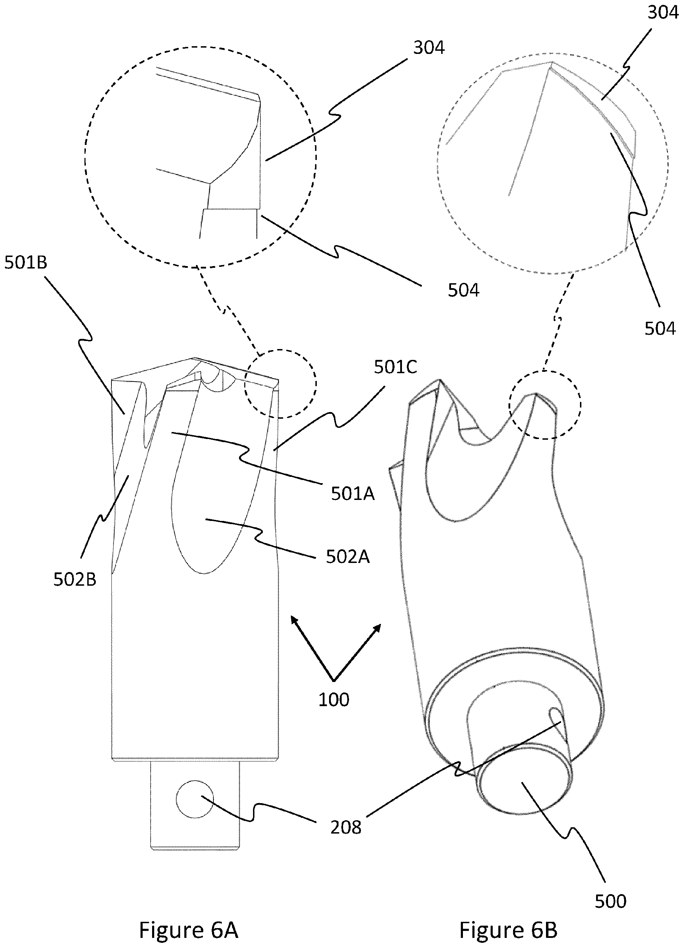

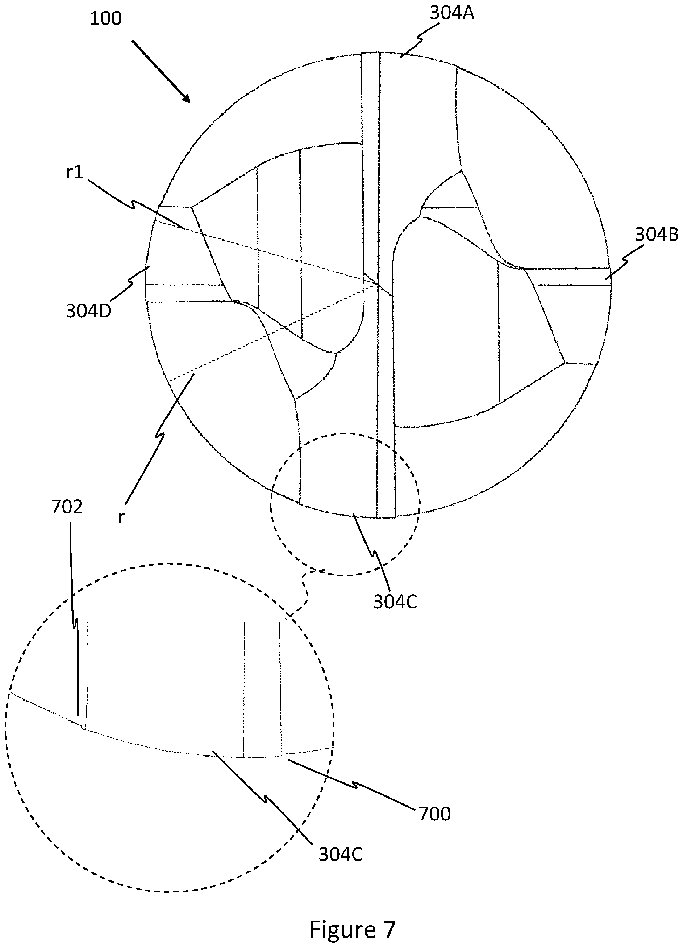

As illustrated in Figure 2, at the distal end, about the circumference of the inner cutting head 106, each cutting protrusion 501 comprises a stepped widening 304, or radial extension, as best seen in Figures 6A, 6B and 7. The stepped widening 304 increases a radius of the inner cutting head 106, as best seen in the enlarged portion of the distal end view of Figure 7. The effect of the stepped widening 304 is discussed in more detail below.

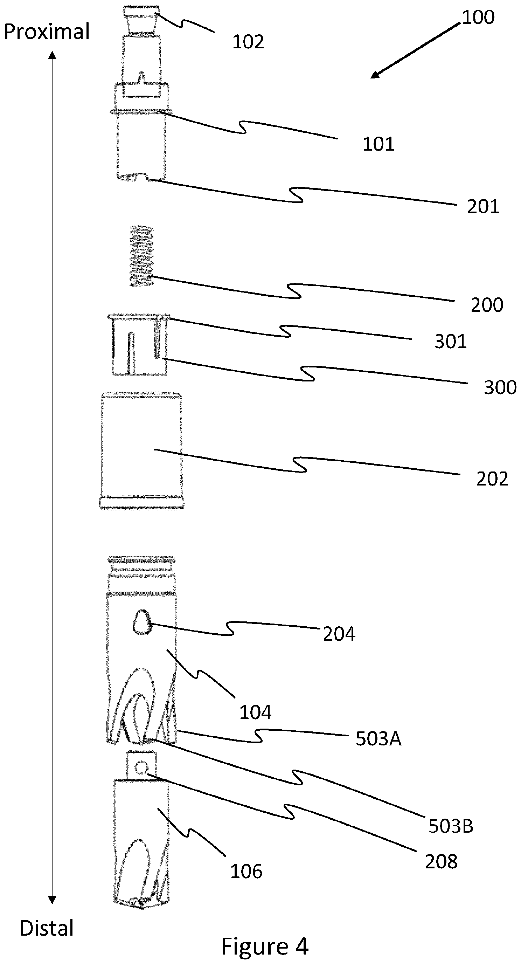

As shown in Figure 4, an exploded view of the perforator 100, the perforator 100 comprises, from a proximal end to a distal end, the drive shaft 101, a spring 200 receivable within the drive shaft 101 and biasing the inner cutting head 106 in a distal direction, away from the drive shaft 101, a spring casing 300, a cylindrical housing 202, the outer cutting head 104, and the inner cutting head 106.

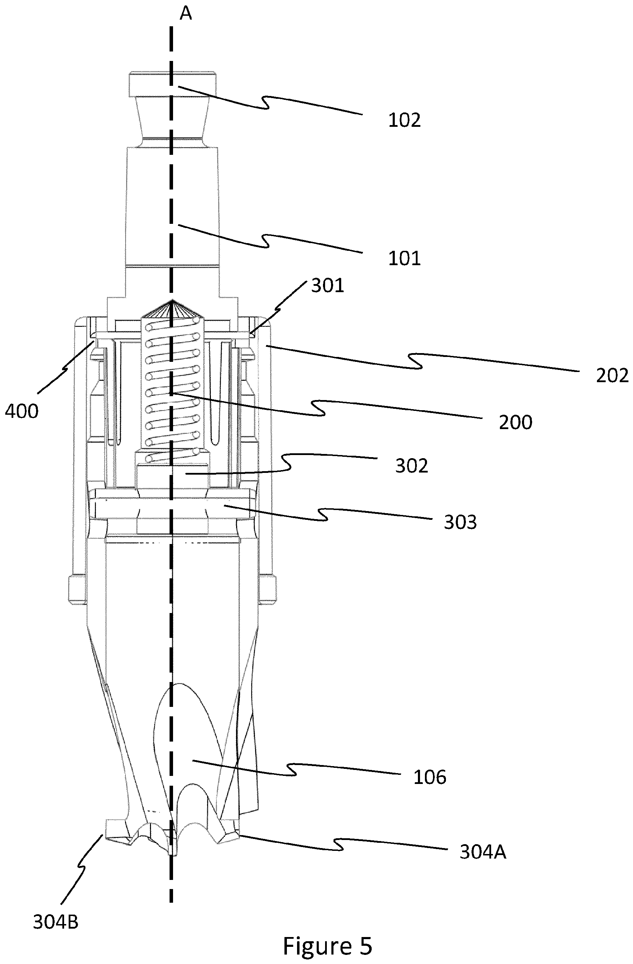

A distal portion of the drive shaft 101, the spring 200, the spring casing 300, a proximal portion of the outer cutting head 104 and a proximal portion of the inner cutting head 106 are provided within the cylindrical housing 202, as shown in the cross section of Figure 5. A distal end of the drive shaft 101 comprises a drive surface 201 configured to engage with a drive pin 303 (shown in Figure 5) of the inner cutting head 106 when the inner cutting head 106 is in the proximal, cutting, position. In particular, the drive pin 303 is provided through an opening 208 in a proximal end portion of the inner cutting head 106, so that it projects, radially, from the opening 208.

The outer cutting head 106 is provided with a triangular slot 204, into which the drive pin 303 extends. As the inner cutting head 106 is pressed against bone in use, a force is applied which moves the inner cutting head 106 against the biasing spring 200 until the drive pin 303 in the inner cutting head engages with the drive surface 201 at the distal end of the drive shaft 101.

In this proximal position, the drive pin 303 also extends into the triangular slot 204 in the wall of the outer cutting head 106 and engages with the apex of the triangular slot 204, so that rotational force is transmitted from the drive shaft 101 via the drive surface 201 to the drive pin 303 and thus the inner cutting head 106 as well as the outer cutting head 104, and both cutting heads 104, 106 rotate together with the drive shaft 101. As long as sufficient force is applied to the tip of the inner cutting head 106 to overcome the biasing force of the spring 200 such that the drive pin 303 remains engaged with the drive surface 201 of the drive shaft 101, the rotating drive shaft 101 transmits rotational force to the inner and outer cutting heads 104, 106 so that both cutting heads 104, 106 rotate and the perforator 100 drills through the bone.

As soon as the inner cutting head 106 perforates the inner surface of the cranium, however, force is no longer applied to the inner cutting 106 head by the bone, so the biasing spring 200 urges the inner cutting head 106 forward (into its distal position) and forces the drive pin 303 out of engagement with the drive surface 201 of the drive shaft 101.

As set out above the inner cutting head 106 is provided at a distal end, about its circumference, with a stepped widening 304, or radial extension, which increases a radius of the inner cutting head 106 at the distal end. The widening 304 has rotational symmetry about the axis A.

Because the radius of the portions of the inner cutting head 106 is greater where the stepped widening 304 is provided, and smaller where no widening is provided, only the portion of the side wall of the inner cutting head 106 provided with the widening 304, or radial extension, is configured to engage with the bone tissue defining the boundary of the hole being cut in the bone tissue.

Prior art perforators, such as the perforator 10 shown in Figure 1, have a large contact area between the side wall of the inner cutting head 12 and the bone tissue defining the boundary of the hole being cut by the inner cutting head 12. This is because the entire side wall of the (cutting protrusions of the) inner cutting head 12 which projects from the outer chipping head 14 when the inner cutting head 12 is in the proximal, cutting, position, will be in contact with the bone tissue defining the hole being cut. In other words, in prior art perforators, the entire substantially cylindrical side wall portion of the cutting head forms an "engaging side wall portion".

On the other hand, because of the widening portions 304A-304D forming the widening 304 having rotational symmetry about rotational axis A as best shown in Figure 7, only the portion of the side wall of the inner cutting head 106 provided with the widening portions 304A-304D (the engaging side wall portion) engages with the wall of bone tissue defining the boundary of the hole, thus reducing the contact area between the bone tissue and the (side walls of the) inner cutting head. In other words, the portion of the cylindrical side wall of the inner cutting head 106, i.e. the side wall portion associated with the cutting protrusions, which is not provided with the widening portions 304A-304D in use is not in contact with the bone tissue defining a periphery of the hole.

Because the widening portions 304A-304D are distributed substantially uniformly (or evenly) around the circumference of the inner cutting head 106, the widening 304 substantially has rotational symmetry about the axis A.

By reducing the contact area between the inner cutting head 106 and the bone tissue, the inner cutting head 106 may be less likely, when compared to prior art perforators, to become jammed in the hole being cut, and thus there is a reduced risk of the cutting heads 104, 106 not decoupling from the drive shaft 101 immediately when the inner cutting head 106 penetrates e.g. the skull. Both the inner and outer cutting heads 104, 106 thus cease to rotate immediately, to prevent damage to the underlying tissue, e.g. the dura mater.

A thickness of each of the widening portions 304A-304D is about 0.025 mm, so that radius r1 is greater than the radius of the remaining portion of the inner cutting head 106 by about 0.025 mm.

A length of each of the widening portions 304A-304D is about 0.8 mm, extending from a distal end of the inner cutting head 106 in a proximal direction.

It is noted that although four cutting protrusions and four widening portions 304A-304D are shown in this example, an inner cutting head 106 may comprise any suitable number of cutting protrusions and widening portions.

As shown in Figure 5, the cylindrical housing 202 is provided, internally, with an annular lip 400, with which an annular flange 301 of the spring casing 300 engages. Further, as also shown in Figure 5, the proximal end portion 302 of the inner cutting head 106, which includes the opening 208 in which the drive pin 303 is provided, engages with the biasing spring 200 via a flat proximal end surface 500 shown in Figure 6B.

As shown in Figures 6A and 6B, between the four cutting protrusions 501A, 501B..., the distal end portion of the inner cutting head 106 is provided with cutting edge flutes 502A, 502B... configured to carry cut material away from a distal tip of the inner cutting head 106. As shown in Figure 6B, the widening portion 304 is defined by a step 504 forming the stepped widening portion 304.

The circumferential limits of each widening portion 304A-304D are defined by circumferential steps 700, 702, as shown in Figure 7. Each widening portion 304A-304D has a circumferential extent of about 25 degrees about the circumference of the inner cutting head, the circumferential extent being defined by the circumferential steps 700 and 702. Although an example cutting edge profile is shown in the distal end view of Figure 7, the invention may be applied to a wide range of cutting heads.

For example, as best seen in Figures 2 and 3, each of a plurality of cutting protrusions 503A, 503B of the outer cutting head 104 also comprise stepped widening portions 505A forming part of a, rotationally symmetrical, widening about the circumference of (a distal end of) the outer cutting head 104. In the same manner as for the inner cutting head 106, the widening increases a radius of the outer cutting head 104, defining a radius of the hole being cut by the outer cutting head.

As the stepped widening portions 505A are provided at a distal end of the outer cutting head, the side wall portions of the outer cutting head 104 which are proximal of the stepped widening portions 505A and have a radius smaller than the radius of the outer cutting head 104 where the stepped widening portions 505A are provided, are not in contact with the bone tissue defining the boundary of the hole being cut. Thus, a contact area between the outer cutting head 104 and the bone tissue is reduced, which may result in less drive power being required to rotate the outer cutting head 104, reducing wear on the coupling mechanism. It may also reduce the force required for removing the perforator from the skull once a hole has been drilled, and may further result in reduced heat generation during drilling.

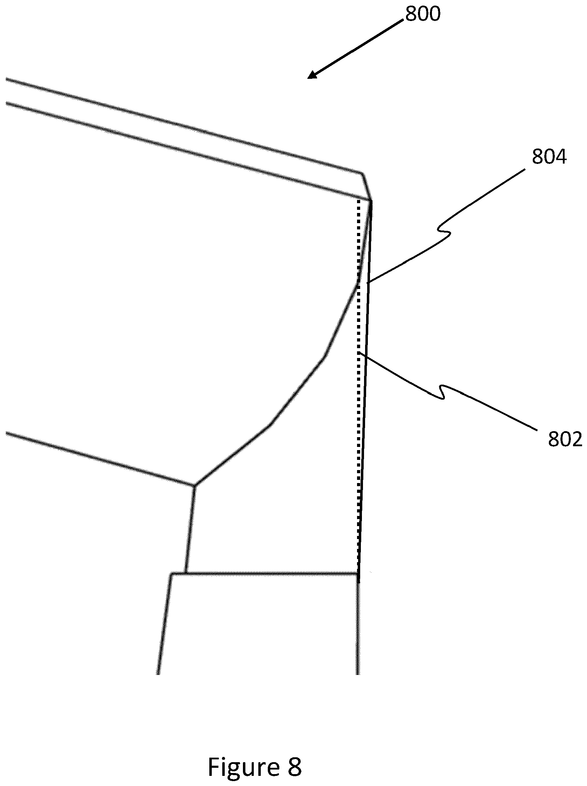

A partial side view of an alternative example of the inner cutting head is shown in Figure 8. Instead of the step 504 and the stepped widening 304, the inner cutting head 800 may have a taper 804, as indicated by the line 802, forming the widening portion. Along the taper 804, a radius of the inner cutting head 800 increases, in a distal direction, to a maximum radius r1. The dotted line in Figure 8 is included to highlight the difference between the radius of a substantially cylindrical cutting head (and thus, the radius of portions of the cutting head 800 where no taper 804 is provided), and the radius of the inner cutting head 800 having a tapered widening (and thus, the radius of the portions of the inner cutting head 800 where the taper 804 is provided).

The effect of the taper 804 may be very similar or identical in reducing a contact area between the side wall of the inner cutting head 800, more precisely the substantially cylindrical side wall portions associated with the cutting protrusions, and the bone tissue as the effect of the stepped widening/step 504 in reducing a contact area between the side wall of the inner cutting head 106, more precisely the substantially cylindrical side wall portions associated with the cutting protrusion, and the bone tissue. As a taper may be provided for each cutting protrusion/around the distal cutting edges, the taper(s) may form a tapered widening, so that the distal end portion of the inner cutting head of this alternative embodiment is substantially frustoconical.

It is noted that the outer cutting head 104 may, instead of the stepped widening 505A shown in Figure 3, have a taper, so that the distal end portion of the alternative outer cutting head is substantially frustoconical.

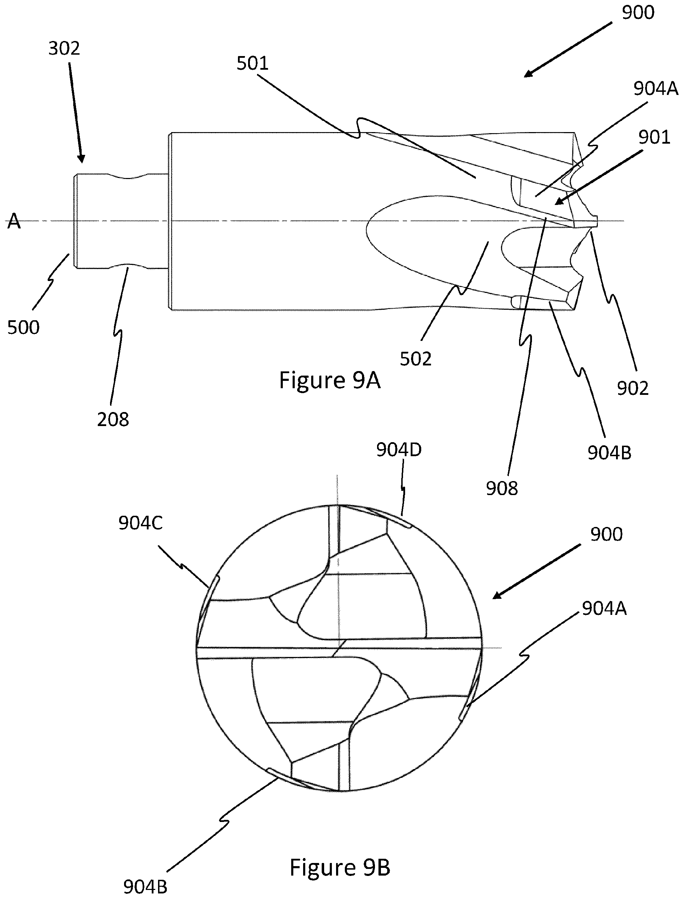

A further example of an inner cutting head 900 is shown Figure 9A. This inner cutting head 900 is similar to a drill bit, in that it has a helical groove 901 extending in a proximal direction along the rotational axis A. The inner cutting head 900 has a (distal) drilling tip 902 or point, and, adjacent each of the flutes 904A, 904B, 904C, 904D of the helical groove 901, the inner cutting head 900 comprises a margin 908. Each narrow margin 908 has a width of about 1 mm. The margin 908 is essentially a portion of the cutting head which is not cut away, meaning that it defines the largest radius r1 of the inner cutting head 900.

At a proximal end, the inner cutting head 900 comprises a proximal end portion 302, which includes an opening 208 in which a drive pin 303 (not shown) is provided, and which engages with the biasing spring 200 via the flat proximal end surface 500, as shown in Figure 6B for inner cutting head 106.

In this manner, only the margin 908 makes up an engaging side wall portion of the inner cutting head 900, while the majority of the side wall portion, and in particularly the majority of the side wall portion of each cutting protrusion, is not in contact with the bone tissue being cut because it has a smaller (and varying) radius than the inner cutting head 900 portion where the margin 908 is provided. In particular, where the flutes 904A...904D of the helical groove 901 are provided, the side wall of the inner cutting head 900 is not in contact with the bone tissue being cut.

As shown in the distal end view of Figure 9B, the cutting geometry of the inner cutting head 900 may be substantially similar to the cutting geometry of inner cutting head 106. The inner cutting head 900 comprises four flutes 904A, 904B, 904C, 904D forming the helical groove 901.

As shown in Figure 9A, the margin(s) 908 and helical groove 901 do not extend to a proximal end of the inner cutting head. Instead, they may be provided, only, mostly, or at least partially, in the portion of the inner cutting head 900 which is configured to project from the outer cutting head when the inner cutting head 900 is in the proximal position and/or in the distal position.

Like inner cutting head 106, between the cutting protrusions 501, the distal end portion of the inner cutting head 900 is provided with cutting edge flutes 502 configured to carry cut material away from distal tip 902.

It is noted that although four cutting protrusions and four flutes 904A, 904B, 904C, 904D of the helical groove 901 are shown in this example, an inner cutting head 900 may comprise any suitable number of cutting protrusions, flutes, and margins.

An outer cutting head may be provided with the helical groove design shown in Figures 9A and 9B for the inner cutting head 900.

## Claims

1. A perforator for drilling bone tissue, comprising:
a drive shaft with a rotational axis A;
an inner cutting head with the same rotational axis A, configured to cut a substantially cylindrical hole; and
a coupling mechanism for coupling the drive shaft and the inner cutting head, wherein the inner cutting head comprises an engaging side wall portion having a radius r1, the radius r1 being a maximum radius of the inner cutting head so that radius r1 defines a radius of a hole the inner cutting head is configured to cut, and a further side wall portion having a radius which is smaller than radius r1.

2. A perforator according to claim 1, wherein the radius r1 is greater than the radius of the further side wall portion by about 0.005 mm to about 5 mm, or about 0.01 mm to about 1 mm, or about 0.015 mm to about 0.5 mm, or about 0.02 mm to about 0.1 mm, or about 0.025 mm to about 0.05 mm.

3. A perforator according to claim 1 or 2, wherein the inner cutting head further comprises a widening forming at least a portion of the engaging side wall portion; optionally wherein the widening:
has rotational symmetry about the rotation axis A;
has a length, along the rotational axis A, of less than about 5 mm, or less than about 2 mm, or less than about 1 mm, further optionally of about 0.1 mm to about 1 mm, or about 0.5 mm to about 0.9 mm, or about 0.8 mm; and/or
comprises at least one of: a stepped widening, and a tapered widening.

4. A perforator according to any preceding claim, wherein the engaging side wall portion is arranged distally of the further side wall portion.

5. A perforator according to any preceding claim, wherein the inner cutting head, at a distal end, further comprises a plurality of cutting protrusions, and optionally wherein each of the plurality of cutting protrusions comprises a widening portion forming a part of the, or a, widening, preferably of the, or a, widening having rotational symmetry about the axis A.

6. A perforator according to any preceding claim, wherein the inner cutting head further comprises:
a helical groove extending helically around the inner cutting head along the rotational axis A; and
a margin forming at least a portion of the engaging side wall portion, and optionally wherein the margin has a width of less than about 3 mm, or less than about 2 mm, or less than about 1 mm.

7. A perforator according to any preceding claim, wherein at least a portion of the inner cutting head is substantially frustoconical.

8. A perforator according to any preceding claim,
wherein the inner cutting head is displaceable with respect to the drive shaft along the rotational axis between a distal position, in which the inner cutting head is not driveable by the drive shaft, and a proximal position, in which the coupling mechanism couples the drive shaft and the inner cutting head to transmit rotational motion from the drive shaft to the inner cutting head,
the perforator optionally further comprising an outer cutting head arranged coaxially around the inner cutting head, in which a cutting head coupling mechanism is provided, the cutting head coupling mechanism being configured to couple the inner cutting head and the outer cutting head when the inner cutting head is in the proximal position so that rotational motion is transmitted from the inner cutting head to the outer cutting head,
further optionally wherein the coupling mechanism comprises the cutting head coupling mechanism.

9. A perforator according to claim 8, wherein the perforator is configured so that when the inner cutting head is in the proximal position, a distal end portion of the inner cutting head projects distally beyond a distal end of a, or the, outer cutting head, and wherein the distal end portion comprises at least a portion of each of the engaging side wall portion and the further side wall portion,
optionally wherein the further side wall portion makes up at least 10 % of a side wall of the distal end portion of the inner cutting head projecting distally beyond the distal end of the outer cutting head when the inner cutting head is in the proximal position, further optionally wherein the further side wall portion makes up at least 20%, at least 30%, at least 40%, at least 50%, at least 60%, at least 70%, or at least 80%, or at least 90%, or at least 95%, or at least 99%, of the projecting side wall.

10. A perforator according to claim 8 or 9,
wherein the perforator is configured so that when the inner cutting head is in the proximal position, the engaging side wall portion and the further side wall portion of the inner cutting head extend distally beyond a distal end of a, or the, outer cutting head; and/or
wherein a, or the, outer cutting head comprises an outer cutting head engaging side wall portion having a radius r3, the radius r3 being a maximum radius of the outer cutting head so that radius r3 defines a radius of a hole the outer cutting head is configured to cut, and an outer cutting head further side wall portion having a radius which is smaller than radius r3.

11. An inner cutting head configured to cut a substantially cylindrical hole into bone tissue, for a perforator for cutting bone tissue according to any of the preceding claims, comprising:
an engaging side wall portion having a radius r1, the radius r1 being a maximum radius of the cutting head so that radius r1 defines a radius of a hole the cutting head is configured to cut; and
a further side wall portion having a radius which is smaller than radius r1,
optionally further comprising: a coupling mechanism portion configured to engage a corresponding coupling mechanism portion of a drive shaft of a perforator when the cutting head is in a proximal position along a rotational axis of the drive shaft, to transmit rotational motion from the drive shaft to the inner cutting head.

12. An inner cutting head according to claim 11, wherein the radius r1 is greater than the radius of the further side wall portion by about 0.005 mm to about 5 mm, or about 0.01 mm to about 1 mm, or about 0.015 mm to about 0.5 mm, or about 0.02 mm to about 0.1 mm, or about 0.025 mm to about 0.05 mm.

13. An inner cutting head according to claim 11 or 12, further comprising a widening forming at least a portion of the engaging side wall portion, optionally wherein the widening:
has rotational symmetry about the axis A;
is arranged at a distal end portion of the inner cutting head, so that the engaging side wall portion is arranged distally of the further side wall portion; has a length, along a rotational axis A of the cutting head, of less than about 5 mm, or less than about 2 mm, or less than about 1 mm, optionally of about 0.1 mm to about 1 mm, or about 0.5 mm to about 0.9 mm, or about 0.8 mm; and/or
comprises at least one of: a stepped widening, and a tapered widening.

14. An inner cutting head according to claim 11, 12, or 13, wherein the inner cutting head, at a distal end, comprises a plurality of cutting protrusions, and preferably each of the plurality of cutting protrusions comprises a widening portion forming a part of the, or a, widening, preferably of the, or a, widening having rotational symmetry about the axis A.

15. An inner cutting head according to any of claims 11 to 14, wherein the inner cutting head further comprises:
a helical groove extending along the rotational axis A; and
a margin forming at least a portion of the engaging side wall portion, and
preferably wherein the margin has a width of less than about 3 mm, or less than about 2 mm, or less than about 1 mm.
